(19) Europäisches Patentamt
European Patent Office
Office européen des brevets

(11) Publication number: **0 161 324**
**B1**

(12) # EUROPEAN PATENT SPECIFICATION

(45) Date of publication of patent specification: **25.11.87**

(51) Int. Cl.$^4$: **A 61 B 6/03,** A 61 B 6/00, G 01 T 1/29

(21) Application number: **84105458.8**

(22) Date of filing: **14.05.84**

(54) **Quantum-counting Radiography method and Apparatus.**

(43) Date of publication of application:
**21.11.85 Bulletin 85/47**

(45) Publication of the grant of the patent:
**25.11.87 Bulletin 87/48**

(84) Designated Contracting States:
**DE FR GB**

(56) References cited:
**DE-A-3 237 773**
**FR-A-2 306 445**
**GB-A-2 017 295**
**US-A-3 937 965**

**IEEE TRANSACTIONS ON NUCLEAR SCIENCE, vol. NS-27, no. 1, February 1980, pages 252-257, IEEE, New York, US; Y. NARUSE et al.: "Multichannel semiconductor detectors for X-ray transmission computed tomography"**

(73) Proprietor: **Matsushita Electric Industrial Co., Ltd.**
**1006, Oaza Kadoma**
**Kadoma-shi Osaka-fu, 571 (JP)**

(72) Inventor: **Baba, Sueki**
**19-16, Izumi-cho 2-chome**
**Suita City 564 (JP)**
Inventor: **Yamamoto, Osamu**
**9-2, Keihan-kitahondouri**
**Moriguchi City 570 (JP)**
Inventor: **Yamashita, Tadaoki**
**16-1, Inda-cho**
**Hirakata City 573 (JP)**
Inventor: **Tsutsui, Hiroshi**
**39-18, Hashimoto-kurigatani**
**Yawata City 614 (JP)**

(74) Representative: **Patentanwälte Kirschner & Grosse**
**Herzog-Wilhelm-Strasse 17**
**D-8000 München 2 (DE)**

**EP 0 161 324 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the invention

1. Field of the invention

The present invention relates generally to a radiography and apparatus for carrying out the radiography and particularly concerns quantum-counting radiography suitable for diagnosis or non-destructive examination and apparatus for carrying it out.

2. Description of the prior art

Hitherto, radiographic recording examination or observation with X-ray, or the like treating high energy radiation, silver salt photographic film or image intensifier of the like apparatus have been used, and also other various proposals are made for higher quality image productions.

Thermo-luminescence film (for instance, disclosed in the Japanese examined patent publication Sho 55—47720) and method of using optical stimulated luminescence film (disclosed in the Japanese unexamined patent publication Sho 55—15025) have proposed such technologies wherein high sensitivity and wide dynamic ranges are expected, but these conventional proposals fail to attain instantaneous reproduction of picture. As another system, CT/T Scout View System for obtaining two-dimensional image of the objective body which utilizes radiation detector array of computed tomography apparatus, wherein the objective body is conveyed in relation to the radiation detector array, has been manufactured and sold by General Electric Company Ltd. of United States. The system comprises xenon gas detector of 511 channels as the radiation detector array for reading out electric charge corresponding to radiation intensity of each detector to produce image. However, the system has poor sensitivity of the detectors and accordingly the object body have to receive large radiation exposure, and it was impossible to improve resolution because of loss of sensitivity when the detector is adapted to small size.

Summary of the invention

Accordingly, the purpose of the present invention is to propose an improved radiography, which has high sensitivity as well as high resolution realized in an instantaneous production of the two dimensional image.

The quantum-counting radiography method in accordance with the present invention comprises the steps of:

emitting a radiation from a radiation source,

receiving radiation which has penetrated through a body by a linear array of radiation sensitive elements thereby to produce signals containing two-dimensional radiographic information, and

changing the relative spatial relation between said radiation source and said radiation sensitive elements on the one hand and said body on the other hand,

characterized in that

the radiation sensitive elements are constituted by semiconductor elements to simultaneously receive said radiation and detect the number of quanta of radiation received, to produce corresponding pulses, the output pulses of said respective radiation sensitive elements are amplified by corresponding pulse amplifiers and counted by subsequent pulse counters connected thereto, to produce corresponding pulse rate number data,

the pulse rate number data from the pulse counters are given to memory means,

then, after said changing said relative spatial relation between said radiation source and said radiation sensitive elements on the one hand and said body on the other hand, the above-mentioned steps are sequentially repeated with cooperative gradual changing of the relative spatial relation, thereby to produce a two-dimensional array of pulse rate number data from which a graded image may be formed.

Also the apparatus for quantum-counting radiography in accordance with the present invention comprises

a radiation source for emitting radiation,

a linear array of radiation sensitive elements for receiving radiation which has penetrated through a body thereby to produce signals containing radiographic information,

means for changing the relative spatial relation between said radiation source and said radiation sensitive elements on the one hand and said body on the other hand,

characterized in that

said radiation sensitive elements are constituted by semiconductor elements to simultaneously receive said radiation rays, and detect the number of quanta of radiation received, to produce corresponding pulses,

the quantum counting radiography apparatus comprising

pulse amplifiers respectively connected to corresponding ones of the radiation sensitive elements,

pulse counters respectively connected to corresponding ones of the pulse amplifiers to issue corresponding pulse rate number data therefrom,

memory means for storing said pulse rate number data, and

controlling means for changing said relative spatial relation, thereby to produce a two-dimensional array of pulse rate number data from which a graded image may be formed.

For instance, the above-mentioned radiation source emits the radiation, for instance, X-ray of at least a fan-shape emission, and the radiation sensitive element to receive the radiation is disposed in a line or in an arc shape, and the objective body such as human body or some machine to be non-destructively examined. The radiation sensitive element is made of some semiconductor. Appropriate semiconductor material for the radiation sensitive element is CdTe and GaAs. Radiation is read out by being converted into discrete pulses, and is amplified by pulse amplifiers and the amplified pulses are counted by pulse counters. That is for each radiation sensitive element, one pulse amplifier and one pulse counter are provided thereby to simultaneously count amount of radiation incident to a multiplicity of radiation sensitive element array. The counted pulse numbers per predetermined unit time, namely, pulse rates are sent to respective addresses of a memory. Then, the set of the radiation source and the array of the radiation sensitive elements is displaced with respect to the objective body, for instance, continuously, or step by step with a small displacement. And after moving to a new position, the same procedures are sequentially repeated many times. Thus two-dimensional information of radiography with respect to the objective body is obtained.

By using of the semiconductor material as the radiation detector together with the below-mentioned features, a drastically high sensitivity in comparison with the conventional system is obtainable. The semiconductor detector element issues one electric pulse for one quantum radiation absorbed by the semiconductor detector, accordingly in principle there is no other method to give higher sensitivity than this. This invention can attain high sensitivity by providing a number of minutely configurated semiconductor radiation sensitive element and by counting the high speed pulse train parellelly at the same time by using a number of pulse amplifiers and a number of pulse counters, thereby attaining high sensitivity as well as high speed detection which can reduce radiation exposure.

Furthermore, the present invention can achieve a high dynamic range, because pulse counting is adopted for the radiation detection, therefore by taking advantage of the large dynamic range, finding of delicate difference of absorption between various soft tissues of human body, for instance, between muscle and fat becomes possible. Thus extensive use for detecting cancer part becomes possible.

Brief description of the drawing

Figure 1 is a perspective view showing outline configuration of the quantum counting radiography of the present invention.

Figure 2 is a perspective view showing a set of array of semiconductor detector elements, together with an array of pulse amplifier and an array of pulse counter.

Figure 3 is a circuit diagram of a semiconductor detector element showing generation of pulse by receiving one quantum of the radiation.

Figure 4(a) is a schematical plan view of the radiation sensitive element array.

Figure 4(b) is front view of the radiation sensitive array of Figure 4(a).

Figure 5 is a graph showing relations between the thickness of one semiconductor element body of the radiation sensitive element and pulse width of the output signal therefrom.

Figure 6 is a graph showing relations between the thickness of one semiconductor element body of the radiation sensitive element and absorbing rate of the element for an X-ray of 60 keV.

Figure 7(a) is a schematic plan view of another embodiment.

Figure 7(b) is a sectional front view of the radiation sensitive array of Figure 7(a).

Figure 8 is a perspective view showing incident directions of the radiation ray to the radiation sensitive element.

Figure 9 is a graph showing stability of relative sensitivity of the semiconductor detector element.

Figure 10 is a graph showing voltage dependency of the capacitance of radiation sensitive element.

Figure 11 is a circuit diagram of a concrete embodiment of circuit to be connected to the semiconductor element of the array.

Figure 12 is a circuit diagram of one example of low impedance type preamplifier 17 of Figure 11.

Figure 13(a) is a plan view showing location of slit number to be used in an actual radiographic system.

Figure 13(b) is a side view showing the location of the slit member 22 in the system of Figure 13(a).

Figure 14(a) is a plan view showing an example of a computor tomography apparatus applying the present invention.

Figure 14(b) is a side view of the embodiment of Figure 14(a).

Description of preferred embodiment

Figure 1 is a perspective view of one example of principle wherein a radiation source, for instance, of an X-ray source 23, and an array of radiation sensitive elements 2, such as CdTe semiconductor detectors are mounted on a frame 24 and a slit 22' is provided in front of the radiation source 23 so as to form the radiation rays in a fan-shape, and the array of radiation sensitive element 2 is disposed on the plane of fan-shape radiation rays to receive them. The objective body 3 such as human body is to be disposed between the slit 22' and the array 2 of the radiation sensitive elements. In this embodiment, the composition consisting of the radiation source 23, the array 2 of radiation sensitive elements, the slit plate 22 having the slit 22' and the arm 24, holding the above-mentioned components in one fixed relation, and the composite construction 100 is slowly or stepwisely with small pitches moved upward or downward.

Figure 2 is an enlarged perspective view of the array of radiation sensitive elements, wherein on a printed circuit substrate 4, an array 5 of detection elements made of semiconductor crystal material is disposed, and thereon electrodes 6 are disposed on respective picture element parts of the semiconductor detector. On the substrate 4, pulse amplifiers 7 are disposed and connected to corresponding electrodes 6 so as to amplify pulse output signal from respective semiconductor detectors. As the semiconductor detector material, various semiconductors may be considered. Hitherto, for such semiconductor detector, silicon crystal or germanium crystal has been used. However, the use of CdTe or GaAs crystal as the material of semiconductor detector is one important feature of the present invention. The single crystal of such material is cut into a bar of about 0.5 mm thickness, about 1 mm width and 10—20 mm length, and the surface is ground smoothly and etching is made thereon. And further thereon, the electrodes 6 and an opposite electrode are formed by known vapour deposition of gold or the like. The front side electrodes 6 are formed in a predetermined number like islands on top surface of the longitudinal crystal bar, and the opposite electrode is formed continuously on the lower surface of the crystal bar which is to contact the surface of the substrate 4. Instead, the opposite side electrode may be formed discretely like islands-shape in the similar manner to the top electrodes 6. A predetermined DC voltage is applied across the top side electrodes 6 and the opposite side electrodes so that a predetermined DC electric fields are generated in the semiconductor detectors. As modified example, comb shaped electrodes may be used. As still other modification, the single crystal cut into individual parts of picture elements which are provided each with a pair of electrodes on opposite sides, may be used to form the array of semiconductor detectors.

As the semiconductor material for the single crystal of the semiconductor detectors, a semiconductor having effective atomic number of over 30 and energy band gap of over 1.3 eV is suitable. Furthermore, the gap between the opposite electrodes of each semiconductor detector, namely the thickness of the semiconductor material, is preferably under 0.5 mm.

In order to improve contrast and resolution of the obtained image, it is naturally preferable that as many pulse number as possible is obtainable from each picture element at each reception of the radiation. In order to achieve this, it is necessary that width of the detected pulses is as small as possible thereby to enable counting of as many pulse number as possible within predetermined time interval. In order to realize the above, it is necessary to decrease the thickness of the semiconductor crystal of the detector element so as to shorten transit time of holes in the semiconductor crystals. Since the pulse width is proportional to square of the thickness, an actually sufficient small thickness is about 0.5 mm, and with a thickness of such or smaller figure, satisfactory good reproduced image of high quality is obtainable by counting $10^5$—$10^6$ counts per picture element. On the other hand, when the semiconductor crystal is thin, absorptional rate of the X-ray is poor. When a material having effective atomic number of over 30 with respect to photoelectric absorption, about 30% absorption rate is obtainable for about 0.5 mm thickness. That is, by selecting the above-mentioned conditions, a satisfactory high sensitivity and high resolution imaging for radiography is obtainable with the semiconductor detectors.

Hereafter, details of high speed pulse counting method of radiation quantum by using the semiconductor detectors is described. The principle of the present invention is elucidated for an example where the radiation is X-rays. The radiation such as X-rays incident on crystal semiconductor of the detectors causes reaction of photoelectric effect or Compton effect in the semiconductor material thereby to generate secondary electrons. The secondary electrons move in the semiconductor crystal and generates electron-hole pairs along their path. Number of such electron-hole pairs are proportional to initial energy of the secondary electrons. As shown in Figure 3, when a strong electric field appears on the semiconductor crystal of the crystal detector by connecting the top side electrode 6 and the bottom side electrode 10 across a potential source Va with a resistance load R in series, the electrons and the positive holes respectively moves towards the bottom electrode 10 and the top side electrode 6 with a high speed, and generates electric charge pulses. Time from reception of the radiation to generation of the secondary electrons and further generation of electron-hole pairs is very short, for instance, about $10^{-12}$ sec. However, since transit time for the electrons and holes, especially the latter moving in the semiconductor crystal takes long time, and accordingly hitherto it was impossible to obtain high speed pulse output of very narrow pulse width. In order to obtain X-ray image of human body, for instance, the composite structure of the X-ray source and the radiation sensitivity element array must be moved within such a short time period of about 10 sec, during which at least more than 100 positions the measuring data from the radiation sensitive element array must be read out, and therefore one measuring time for each detection element must be smaller than 100 m.sec. When the pulse width is broad, number of pulses that can be counted in this measuring interval becomes small, thereby decreasing the dynamic range for the picture element, and also lowering accuracy of counting measurement. Accordingly in order to improve counting accuracy, the pulse width must be made as narrow as possible, namely the pulse must be made high speed pulses. Hitherto, because of this problem the pulse counting measurement has not been utilized, but instead the measurement has been delied on galvanometric method (for instance, see, Y. Naruse et al., IEEE Trans. NS-27(1), p. 252, 1980). Besides, since the galvanometric measuring method measures time-mean value of electric-charge pulses of radiation quantums, its accuracy of the radiation detection of low dose region has been more than two digits smaller than that of the pulse counting method.

One of the important features of the present invention is that in order to measure pulse number, the radiation sensitive element, i.e., semiconductor crystal chips 5, 5 . . . of semiconductor detectors are formed very thin as shown in Figure 4. And on opposite faces of each semiconductor crystal chip 5 a pair of electrodes, namely, top electrode 6 and bottom electrode 10 are formed. The bottom electrode 10 may be formed commonly to all semiconductor crystal chips. By selecting the thickness of the semiconductor crystal chips very thin, transnit times of the electrons and positive holes to the electrodes 6 and 10 are much decreased, thereby very narrow pulses, hence high speed pulse train is obtainable. This point is described further in detail with reference to Figure 3. Electron-hole pairs generated by radiation quantum receive effect of electric field applied to the semiconductor crystal through the application of potential from the electrodes 6 and 10. As a result, the electrons are attracted to anode 10 and the positive holes are attracted to cathode 6, respectively. Therefore, pulse current is generated in the external circuit which comprises the resistor R and the potential source Va. And the pulse width t of the pulse signal is given by the following known equation:

$$t=d^2/\mu \cdot V_a \qquad (1),$$

wherein t is pulse time width,

d is distance across two opposite electrodes on the semiconductor crystal chip of the semiconductor detector,

μ is mobility of electrons or positive holes, and

$V_a$ is potential applied across the two opposite electrodes on the semiconductor crystal chip.

According to the above-mentioned equation, pulse time width t is proportional to square of the thickness of the semiconductor crystal chip, accordingly when the thickness is reduced to 1/10, the pulse width is reduced to 1/100.

Now, let us examine actual examples utilizing CdTe which has effective atomic number of $Z_E=49$ and GaAs which has effective atomic number of $Z_E=31$. Hole-mobility $\mu_h$ of CdTe=$\mu_h$=80 cm$^2$/V · sec, and hole-mobility $\mu_h$ of GaAs is $\mu_h$=400. A voltage of 100 V is impressed across the electrode, and pulse width for various thicknesses of semiconductor crystal are plotted as shown in Figure 5. Wherein for 1 mm thickness of the semiconductor crystal chip, pulse width of CdTe is 1 μsec and that of GaAs is 0.25 μsec. When the thickness of the semiconductor crystal chip is 0.5 mm, pulse width of CdTe becomes 0.3 μsec and that of GaAs becomes 0.06 μsec. As is compared, for difference of the thickness of 1 mm and 0.5 mm the pulse width differs by several times. Thus, for the thickness of under 0.5 mm, the pulse width becomes below 0.3 μsec. This pulse width is substantially the narrowest one which can be achieved by actually usable modern circuit technology. Now, provided that pulse integration time for one picture element is 100 m sec, number of 0.1 μsec width pulse in the 100 m sec becomes (100 m sec÷0.1 μsec=) $10^6$. When the pulses are random pulses, error due to statistical fluctuation is $\sqrt{10^6}=10^3$, and measurement error for the picture element becomes 0.1%. It is generally known that differences of diagnosis X-ray absorption coefficient between soft tissue such as muscle, fat and substantial water part are below several percent, and hence, in order to detect such slight difference such measurement accuracy as 0.1% becomes necessary. The above-mentioned statistical fluctuation of incident X-ray requires to hold such accuracy for producing meanfull image.

However, if thickness of the semiconductor crystal chip is decreased, radiation absorbing rate is lowered, thereby to lower radiation sensitivity. Figure 6 shows relation between semiconductor crystal thickness of CdTe and GaAs versus absorbing rate (%) of 60 keV X-ray. In general, effective energy of X-ray used for diagnosis is around 60 keV. When semiconductor crystal thickness is about 0.5 mm, CdTe that has effective atomic number with respect to photoelectric absorption is about 50, and the absorbing rate is about 80% which is sufficiently large. When the thickness is 0.1 mm, the absorption rate becomes about 30% and is almost actually usable. Though GaAs has effective atomic number of about 32, and absorption rate is about 30% for 0.5 mm thickness semiconductor crystal chip, which is smaller than that of CdTe, it is almost actually utilizable. A semiconductor crystal having effective atomic number smaller than 30 has poor X-ray absorption rate, and accordingly it is not actually usable because of too low sensitivity against X-ray.

As summarizing the above, a semiconductor crystal chip used as the radiation sensitive element has sufficient sensitivity for absorption of X-rays used in diagnosis when the chip thickness is around 0.5 mm. Furthermore, when the semiconductor crystal chip is thinner than 0.5 mm, pulse width becomes very narrow.

Figure 7(a) and Figure 7(b) show another embodiment of the present invention wherein Figure 7(a) shows plan view of composite structure of semiconductor crystal elements 5 provided on a substrate 4, wherein on a continuous lengthwise semiconductor crystal chip 5, a number of discrete top side electrodes 6, 6 . . . are formed. This type of the radiation sensitive element can be manufactured more easily than the embodiment of Figure 4(a) and Figure 4(b). The actual configuration of the semiconductor crystal array is not limited to the configurations of the above-mentioned embodiments.

Incident direction of the radiation into the semiconductor crystal detector elements is not limited, since penetration force of the radiation is adjustable. For instance, as shown in Figure 8, a metal plate 15 may be used as a filter to adjust the energy of incident radiation. Such radiation sensitive element array may be used to receive incident light 14 in a direction to receive the radiation directly to the semiconductor crystal chips 5 for radiation to produce image of high accuracy or for radiation of short penetration depth. (Each semiconductor crystal chip 5 constituted one detector element corresponding to one picture element.)

Or alternatively, the radiation may be incident to the semiconductor crystal chips from a direction 14' to come through a metal plate or the like intensity-controlling substance 15. By such selection of the incident direction, intensity of radiation incident to the semiconductor crystal chips 5 may be freely designed, and therefore improvement of sensitivity or resolution may be achieved therewith. In the configuration of Figure 8, the substrate 4 is located so as to prevent the scattered radiation from side face of the semiconductor crystal chips 5, thereby to prevent becoming worse of picture quality due to the scattered of the radiation. The plate 15 may be a shield against radiation which is independent from the substrate 4 provided on opposite sides of the semiconductor crystal chips 5. The substrate 4 as such may be a shield against the radiation. For the material as the shield, those materials liable to make noises for the semiconductor crystal detector is not desirable. For instance, such material as to generate high energy X-rays by incident X-rays or β-rays, such as lead or wolfram, or such material as to become radioactive by incident thermal neutrons, such as, gold, indium or cadmium should not be used. By using pulse height analyzer, various scattered rays or other noise signals induced by incident radiation signal can be removed to some extent, but it is necessary to suppress generation of noise as such, as lower as possible in order to obtain high accuracy image.

Nextly, configuration of the semiconductor detector elements are described. In general, there are PN junction type electrodes and surface barrier type electrodes for semiconductor detector elements. Both of these electrodes are formed by forming high resistance layer on the surface thereby to decrease leak current produced by voltage impressed on the detector, to improve S/N ratio. However, the conventional electrodes of PN junction type and surface barrier type have such shortcomings as liability of making drifts due to accumulation of electron charge at high resistance layer or drifting of sensitive area due to variation of applied voltage or ambient temperature. Accordingly, in the radiation sensitive elements of the present invention, ohmic contact electrodes are adopted as electrodes on both sides of the semiconductor detector elements in order to overcome the above-mentioned shortcomings. In order to utilize ohmic junctions, it is necessary to develop a method for lowering leak current due to impression of bias-voltage.

Provided that, energy given by radiation quantum incident to semiconductor detector element is Eg. average energy to produce one pair of electron-hole pair in the semiconductor detector element is Wg, and pulse width of the output pulse signal is t similar in the equation (1), then maximum output current $i_{max}$ generated by the incident radiation rays is given by the following equation (2):

$$i_{max} = \frac{Eg}{Wg \cdot t} \times 1.6 \times 10^{-19} [A] \qquad (2).$$

When, for instance, incident radiation rays are X-rays of Eg=60 KeV, a CdTe crystal chip is used as the semiconductor detector element, impressed across a pair of electrodes formed on both faces of the crystal chip of 0.5 mm thickness, then the pulse width t becomes 0.3 μsec and Wg≑4 eV, accordingly, the above-mentioned equation (2) becomes as follows:

$$i_{max} = 8 \times 10^{-9} [A] \qquad (3).$$

Our experiments show that a satisfactory S/N ratio is obtainable when the leak current is under a value as large as 100 times of the value given by the above-mentioned (3). As a result, specific resistance of the semiconductor material is over $10^8$[Ωcm] for such a material of small hole-mobility as CdTe, and on the other hand for such material as GaAs which has larger hole-mobility a satisfactory S/N ratio is obtainable even with adopting ohmic contact electrodes for both electrodes even for the specific resistance of over $10^7$[Ωcm].

The specific resistance can be increased by cooling semiconductor material or compensating the impurity of the semiconductor or the like. However, since it is advantageous to increase the mobility as large as possible, especially the hole-mobility which is the characteristic of the semiconductor in making the pulse detection of radiation quantum, an intensive compensation of the impurity is not preferable. On the other hand, in order to make the radiography apparatus simple, provision of complicated refrigerating apparatus is not preferable. Accordingly, the present invention uses such a material as having specific resistance of over $10^7$ [Ωcm] at room temperature, namely, a semiconductor material having energy band gap of over 1.3 eV. Thus, as materials having effective atomic number of above 30, having energy band gap of over 1.3 eV, and furthermore, having hole-mobility of above 50, the above-mentioned GaAs and CdTe are suitable.

Figure 9 shows stability of relative sensitivity, i.e., relative sensitivity versus time with applied voltage between the two opposite electrodes of crystal detection element of CdTe having ohmic contact electrodes. Figure 10 shows characteristic of applied voltage versus reactive capacitance of the same detection element. Quantum of radiation rays absorbed in the semiconductor crystal element are transduced into electric pulses, which are then given to pulse amplifiers 7, and after amplification therein further given to the corresponding pulse counters, and the counted data are further given to corresponding memories. The pulse amplifier, the pulse counter and the memory are provided for each semiconductor crystal detector, so that radiation rays incident to semiconductor detector elements of the array as shown in Figure 1 can be detected and stored simultaneously. The above-mentioned detections of radiations and storings of the detected and counted information data thereof are successively carried out by stepwisely or gradually moving the set of the array of the semiconductor crystal detectors and the radiation source. The counted data temporarily stored in the memories are read out to outside circuitry in sequence.

Figure 11 shows a circuit diagram of one example of the set for one semiconductor detector element and electric circuits belonging thereto. The circuit comprises a FET amplifier 16 which is for converting high impedance pulse P signal into the low impedance pulse signal. The impedance-converted input pulse signal is then given to the amplifier 17, and then given to a discriminator 17', and then given to the pulse counter 18. The counted data from the pulse counter 18 is temporarily stored in the memory 19, the data of which is to be given to a known memory or a known processor. These circuits are constituted on an IC and connected with the semiconductor detector elements.

In general, in radiation pulse measurement, because of the reason to highly regard uniformity of height of pulse output of respective radiation sensitive elements regardless of capacitances of the respective sensitive elements, hitherto preamplifiers of electric charge amplifier type have been mostly used. For a circuit to count pulses of $10^5$ counts per second, however, such type amplifier needs too much complication in circuitry, and is not suitable for the system wherein a number of sets of the semiconductor detector elements and pulse amplifiers are provided. Accordingly, the quantum counting radiography apparatus in accordance with the present invention has been improved to comprise high gain inverting amplifier having junction FET of low impedance capacitance as input stage, and further comprising as a pulse amplifier shown in Figure 12 a preamplifier of low input impedance type comprising a feedback loop having a resistor and a capacitor for forming frequency dependent feeding back, and a satisfactory result has been obtained.

The semiconductor detector elements of the present invention has very minute configuration, and accordingly capacitance of each element is below 2 pF, and that, the electrodes of the semiconductor detector elements are of ohmic contact electrodes which are not influenced by impressed voltage or ambient temperature, accordingly the semiconductor detector elements have satisfactory performance by using voltage-amplification type preamplifier which has splendid high speed response characteristic. But, since the semiconductor detector element has such very high internal resistance as over $10^7\ \Omega$, the ordinary voltage-amplifier type preamplifier could not result in satisfactory high speed response characteristic. Accordingly, the inventors developed a special preamplifier with which good S/N ratio as well as high speed pulse rate counting can be realized, and the preamplifiers can be made in integrated circuit since the circuit construction is simple.

Figure 12 shows a preferable embodiment which is suitable as the preamplifier shown in Figure 12. When the preamplifiers are made on array-shaped integrated circuit, uniformity of characteristics of junction FET is an important problem. And accordingly, in this example, a constant current source CCS is adopted as drain current source of the J-FET 16, so that dispersion of characteristics of J-FET due to dispersion of the gain of the preamplifier is prevented. The output of the J-FET 16 is amplified by a voltage-amplifier VA and is output through an emitter-follower EF.

In general, it is desirable that number of picture elements of radiography in vertical direction and in horizontal direction should be both more than 500. On the other hand, time required for radiography should be shorter than 10 seconds at longest, and hence, moving of the composite construction of the radiation source and the array of the radiation sensitive elements must be within this range of time. As a result, pulse counting period for one picture element must be under 30 m sec at longest. That is, in order to obtain image of high quality radiography, a dynamic range of over 60 dB is necessary, and hence, counting ability of about $10^5$ pulses per second must be provided. Accordingly, in order to count $10^5$ pulses in 30 m sec, a pulse time resolution power shorter than 0.3 μ sec is necessary. The aforementioned semiconductor detector elements have been developed recently to have the performance of such pulse time resolution.

Sensitivity of the array is determined by effective incident across sectional area and thickness of the sensitive layer, and accordingly the dispersion of sensitivity is substantially determined by accuracy of cutting on the semiconductor crystal detector elements. Therefore, in order to adjust the sensitivity, trimming of the semiconductor crystal element is carried out. But as other method, adjusting of sensitivity in the discriminator part of the pulse amplifier, compensation of count in the counter circuits or compensation in data processing parts can be made. Furthermore, compensation can be made for dispersion of amplitude of the output pulses between a number of the semiconductor crystal detector elements by making sensitivity adjustment in the pulse amplifier or in pulse height level discriminator (i.e., comparator).

Nextly, devicing in radiation emission is described. The devicing is made in order to decrease radiation exposure to the objective body as low as possible. As shown in Figure 13(a) which is a plan view of the quantum counting radiography apparatus in accordance with the present invention, and Figure 13(b) which is the sectional view of the same apparatus, a radiation shield having a slit 22' thereon is disposed between the radiation source 23 and the objective body 21. That is, the radiation shield 22 is fixed on the frame 24, on which the radiation source 23 and the array 20 of the radiation sensitive elements are mounted. As has been described, the semiconductor crystal detector elements of the radiation sensitive elements should be made as thin as possible, for instance under 0.5 mm thick. Accordingly, by accurately disposing the array of the radiation sensitive elements in a manner to receive very thin fan-shaped radiation rays irradiated through the slit 22′, amount of exposure of radiation to the objective body can be made as small as possible. Therefore, the width of the slit 22′ is designed so narrow as 1 mm. And in order to accurately emit the radiation rays through the slit 22′ on the very thin semiconductor crystal chips in the array of the radiation sensitive element, the radiation shield 22 having the slit 22′ is rigidly fixed on the frame 24 together with the radiation source 23 and the array 20. By such construction, the very thin fan-shaped radiation can be received by the array of thin semiconductor crystal chips of the radiation sensitive elements.

As another configuration, an accurate irradiation of radiation rays to the radiation sensitive elements array through the slit 22′, may be made by co-operatively moving the slit 22′ and the array 20 while fixing the radiation beam source 23. In such configuration, accurate control of the array can be made by disposing additional radiation sensitive elements for detecting positional relation preferably on both ends of the array. Thereby, the moving of the array is servomechanically controlled by utilizing the position detection information from the additional radiation sensitive elements.

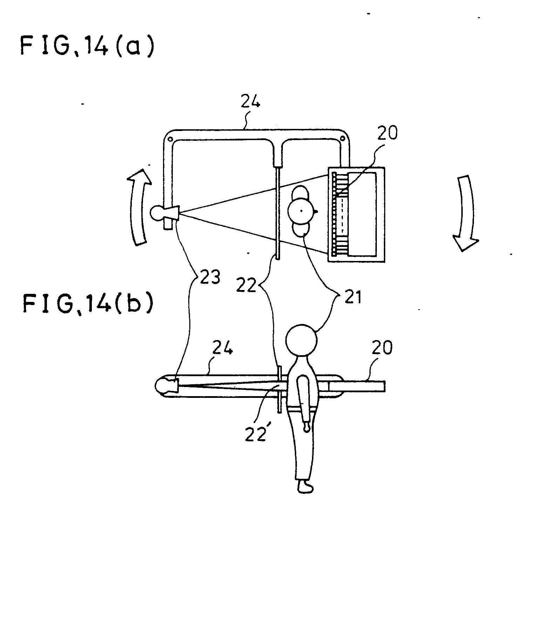

Figure 14(a) which is a plan view and Figure 14(b) which is a side view of another embodiment show a configuration that the radiation beam source 23 and the array 20 of the radiation sensitive elements are moved on a plane of the fan-shaped radiation rays. This configuration, as is known, can produce tomographic image information.

The above-mentioned embodiments revealed that, by using 500 bit array of radiation sensitive elements used to actually receive radiation rays of fan shape of 60 KeV and 1 mR X-rays, a clear X-ray image was obtained. And the obtained X-ray image has several tens times higher sensitivity than the conventional X-ray photograph using silver salt or conventional pulse counting or electric charge or galvanic apparatus using an array of xenon discharge tubes. Furthermore, the quantum counting radiography in accordance with the present invention can produce image immediately after emitting of the radiation contrasted to the conventional silver salt photographic method or thermal luminescence method which takes a considerable time for developing and fixing. The quantum counting radiography in accordance with the present invention can of course be applied to compute tomography. Experiments showed that when the radiographic image obtained by using $10^6$ count pulses per one picture element, satisfactory contrast is obtainable thereby enabling delicate difference of soft tissues such as cancer tissue in the surrounding normal tissues, which has been impossible with the conventional silver salt photography method. Experiments showed that a cancer focus of a size of within 1 cm can be detected in accordance with the present invention.

**Claims**

1. A quantum-counting radiography method comprising the steps of:

emitting radiation from a radiation source, receiving radiation which has penetrated through a body by a linear array of radiation sensitive elements thereby to produce signals containing radiographic information, and

changing the relative spatial relation between said radiation source and said radiation sensitive elements on the one hand and said body on the other hand,

characterized in that

said radiation sensitive elements are constituted by semiconductor elements to simultaneously receive said radiation and to detect the number of quanta of radiation received, to produce corresponding pulses,

the output pulses of said respective radiation sensitive elements are amplified by corresponding pulse amplifiers and counted by subsequent pulse counters connected thereto, to produce pulse rate number data, said pulse rate number data from said pulse counters are given to memory means,

then, after said changing of said relative spatial relation between said radiation source and said radiation sensitive elements on the one hand and said body on the other hand, the above-mentioned steps are sequentially repeated with cooperative gradual changing of said relative spatial relation, thereby to produce a two-dimensional array of pulse rate number data from which a graded image may be formed,

2. A quantum-counting radiography method in accordance with claim 1, wherein

said semiconductor elements are made of a semiconductor material having effective atomic number of above 30, and

said semiconductor elements are disposed substantially linear on a substrate and each provided with a pair of corresponding plural electrodes on opposite faces of each semiconductor element with 0.1—0.5 mm distance between the pair of electrodes,

thereby to constitute said linear array of radiation sensitive elements.

3. A quantum-counting radiography method in accordance with claim 1, wherein

said semiconductor elements are made of a semiconductor material having energy band gap of above 1.3 eV.

4. A quantum-counting radiography method in accordance with claim 1, which further comprises

a radiation shield for said radiation having a slit for restricting the radiation rays to a fan-shaped rays lying on a plane to contain said linear array of radiation sensitive elements.

5. A quantum-counting radiography method in accordance with claim 4, wherein

said radiation source, said radiation shield and said linear array of radiation sensitive elements are mounted in a composite construction of fixed spatial relation, which is held movably in relation to said body.

6. A quantum-counting radiography method in accordance with claim 4, wherein

said radiation shield is disposed between said radiation source and said body, and

said radiation shield and said linear array are moved in such an operative manner that said linear array is on a plane defined by said radiation source and said slit.

7. A quantum-counting radiography method in accordance with claim 1, wherein

radiation pulse counting time of each one of said radiation sensitive element is below 30 m sec.

8. A quantum-counting radiography method in accordance with claim 1, which further comprises

radiation sensitivity adjusting means for adjusting or compensating at least one part of output of said radiation sensitive elements.

9. A quantum-counting radiography method in accordance with claim 1, which further comprises

radiation sensitivity adjusting means which is constituted by computing means connected to at least a part of said radiation sensitive elements for adjustment or compensation of outputs of said radiation sensitive elements.

10. A quantum-counting radiography method in accordance with claim 1, wherein

said semiconductor elements are made with CdTe.

11. A quantum-counting radiography method in accordance with claim 1, wherein

said semiconductor elements are made with GaAs.

12. A quantum-counting radiography apparatus comprising:

a radiation source for emitting radiation,

a linear array of radiation sensitive elements for receiving radiation which has penetrated through a body thereby to produce signals containing radiographic information,

means for changing the relative spatial relation between said radiation source and said radiation sensitive elements on the one hand and said body on the other hand,

characterized in that

said radiation sensitive elements are constituted by semiconductor elements to simultaneously receive said radiation, and detect the number of quanta of radiation received, to produce corresponding pulses,

said quantum counting radiography apparatus comprising

pulse amplifiers respectively connected to corresponding ones of said radiation sensitive elements,

pulse counters respectively connected to corresponding ones of said pulse amplifiers, to issue corresponding pulse rate number data therefrom,

memory means for storing said pulse rate number data, and

controlling means for changing said relative spatial relation, thereby to produce a two-dimensional array of pulse rate number data from which a graded image may be formed.

13. A quantum-counting radiography apparatus in accordance with claim 12, wherein

said semiconductor elements are made of a semiconductor material having effective atomic number of above 30, and

said semiconductor elements are disposed substantially linear on a substrate and each provided with a pair of corresponding plural electrodes on opposite faces of each semiconductor element with 0.1—0.5 mm distance between the pair of electrodes,

thereby to constitute said linear array of radiation sensitive elements.

14. A quantum-counting radiography apparatus in accordance with claim 12, wherein

said semiconductor elements are made of a semiconductor material having energy band gap of above 1.3 eV.

15. A quantum-counting radiography apparatus in accordance with claim 12, which further comprises

a radiation shield for said radiation having a slit for restricting the radiation rays to a fan-shaped rays lying on a plane to contain said linear array of radiation sensitive elements.

16. A quantum-counting radiography apparatus in accordance with claim 15, wherein

said radiation source, said radiation shield and said linear array of radiation sensitive elements are mounted in a composite construction of fixed spatial relation, which is held movably in relation to said body.

17. A quantum-counting radiography apparatus in accordance with claim 15, wherein

said radiation shield is disposed between said radiation source and said body, and

said radiation shield and said linear array are moved in such an operative manner that said linear array is on a plane defined by said radiation source and said slit.

18. A quantum-counting radiography apparatus in accordance with claim 12, wherein

radiation pulse counting period of each one of said radiation sensitive element is below 30 m sec.

19. A quantum-counting radiography apparatus in accordance with claim 12, which further comprises

radiation sensitivity adjusting means for adjusting or compensating at least one part of output of said radiation sensitive elements.

20. A quantum-counting radiography apparatus in accordance with claim 12, which further comprises

radiation sensitivity adjusting means which is constituted by computing means connected to at least a part of said radiation semiconductor elements for adjustment or compensation of outputs of said radiation sensitive elements.

21. A quantum-counting radiography apparatus in accordance with claim 12, wherein

said semiconductor elements are made with CdTe.

22. A quantum-counting radiography apparatus in accordance with claim 12, wherein

said semiconductor elements are made with GaAs.

**Patentansprüche**

1. Verfahren mit Strahlungsquantenzählung, welches folgende Schritte umfaßt:

Aussenden von Strahlung von einer Strahlungsquelle,

Empfangen von Strahlung, die einen Körper durchdrungen hat, durch eine lineare Anordnung von strahlungsempfindlichen Elementen, um dadurch Röntgendaten enthaltende Signale zu erzeugen,

Verändern der relativen räumlichen Beziehung zwischen den strahlungsempfindlichen Elementen einerseits und dem Körper andererseits, dadurch gekennzeichnet, daß

die strahlungsempfindlichen Elemente von Halbleiterelementen gebildet werden, um gleichzeitig die Strahlung empfangen und die Zahl der Quanten der empfangenen Strahlung feststellen zu können, um entsprechende Impulse zu erzeugen,

die Ausgangssignale der jeweiligen strahlungsempfindlichen Elemente durch entsprechende Impulsverstärker verstärkt und durch damit verbundene, nachgeschaltete Impulszähler gezählt werden, um Impulsratendaten zu erzeugen,

die Impulsratendaten von den Impulszählern an Speichereinheiten abgegeben werden, und daß

dann, nach dem Verändern der relativen räumlichen Beziehung zwischen der Strahlungsquelle und den strahlungsempfindlichen Elementen einerseits und dem Körper andererseits, die obengenannten Schritten sequentiell unter allmählicher Änderung der relativen räumlichen Beziehung wiederholt werden, um dadurch eine zweidimensionale Anordnung von Impulsratendaten, aus denen ein abgestuftes Bild gebildet werden kann, zu erzeugen.

2. Verfahren mit Strahlungsquantenzählung nach Anspruch 1, dadurch gekennzeichnet, daß die Halbleiterelemente aus einem Halbleitermaterial mit einer effektiven Atomzahl über 30 gefertigt sind, und daß die Halbleiterelemente im wesentlichen linear auf dem Substrat angeordnet sind und jedes ein Paar von entsprechenden Mehrfachelektroden auf gegenüberliegenden Seiten jedes Halbleiterbauelementes mit einem Abstand von 0,1—0,5 mm zwischen dem Paar aufweist, um dadurch die lineare Anordnung der strahlungsempfindlichen Elemente zu bewirken.

3. Verfahren mit Strahlungsquantenzählung nach Anspruch 1, dadurch gekennzeichnet, daß die Halbleiterelemente aus einem Halbleitermaterial mit einem Energiebandabstand von über 1,3 eV hergestellt sind.

4. Verfahren mit Strahlungsquantenzählung nach Anspruch 1, weiterhin gekennzeichnet dadurch, daß ein Strahlungsschild für die Strahlung mit einem Schlitz vorgesehen ist, um die Strahlen der Strahlung auf fächerförmige, in einer die lineare Anordnung von strahlungsempfindlichen Elementen enthaltenden Ebene liegende Strahlen zu begrenzen.

5. Verfahren mit Strahlungsquantenzählung nach Anspruch 4, dadurch gekennzeichnet, daß die Strahlungsquelle, der Strahlungsschild und die lineare Anordnung von strahlungsempfindlichen Elementen als Verbundstruktur mit einer festen räumlichen Beziehung aufgebaut sind, die bezüglich des Körpers beweglich gehalten ist.

6. Verfahren mit Strahlungsquantenzählung nach Anspruch 4, dadurch gekennzeichnet, daß der Strahlungsschild zwischen der Strahlungsquelle und dem Körper angeordnet ist und daß der Strahlungsschild und die lineare Anordnung betriebsmäßig so bewegt werden, daß die lineare Anordnung auf einer Ebene liegt, die durch die Strahlungsquelle und den Schlitz definiert ist.

7. Verfahren mit Strahlungsquantenzählung nach Anspruch 1, dadurch gekennzeichnet, daß die Strahlungsimpulszählzeit jedes einzelnen der strahlungsempfindlichen Elemente unter 30 m sec liegt.

8. Verfahren mit Strahlungsquantenzählung nach Anspruch 1, dadurch gekennzeichnet, daß es ferner eine Strahlungsempfindlichkeits-Einstellvorrichtung zum Einstellen oder Ausgleichen von wenigstens einem Teil des Ausgangs der strahlungsempfindlichen Elemente enthält.

9. Verfahren mit Strahlungsquantenzählung nach Anspruch 1, dadurch gekennzeichnet, daß es ferner eine Strahlungsempfindlichkeits-Einstellvorrichtung enthält, die durch einen Rechner gebildet wird, der mit wenigstens einem Teil der strahlungsempfindlichen Elemente verbunden ist, um die Ausgänge der strahlungsempfindlichen Elemente einzustellen oder auszugleichen.

10. Verfahren mit Strahlungsquantenzählung nach Anspruch 1, dadurch gekennzeichnet, daß die Halbleiterelemente mit CdTe hergestellt werden.

11. Verfahren mit Strahlungsquantenzählung nach Anspruch 1, dadurch gekennzeichnet, daß die Halbleiterelemente mit GaAs hergestellt werden.

12. Einrichtung mit Strahlungsquantenzählung, enthaltend:

eine Strahlungsquelle zum Aussenden von Strahlung,

eine lineare Anordnung von strahlungsempfindlichen Elementen zum Empfangen der Strahlung, die einen Körper durchdrungen hat, um Röntgendaten enthaltende Signale zu erzeugen,

eine Vorrichtung zum Verändern der relativen räumlichen Beziehung zwischen der Strahlungsquelle und den strahlungsempfindlichen Elementen einerseits und dem Körper andererseits, gekennzeichnet dadurch, daß

die strahlungsempfindlichen Elemente durch Halbleiterelemente gebildet werden, um gleichzeitig die Strahlung zu empfangen und die Zahl der Quanten der empfangenen Strahlung festzustellen, um entsprechende Impulse zu erzeugen,

wobei die Strahlungsquantenzähl-Vorrichtung enthält:

Impulsverstärker, die jeweils mit entsprechenden der strahlungsempfindlichen Elemente verbunden sind,

Impulszähler, die jeweils mit entsprechenden der entsprechenden der Impulsverstärker verbunden sind, um daraus Impulsratendaten abzugeben,

Speichereinrichtungen zum Speichern der Impulsratendaten, und

Steuereinrichtungen zum Ändern der relativen räumlichen Beziehung, um dadurch einöe zweidimensionale Anordnung von Impulsratendaten zu erzeugen, aus der ein abgestuftes Bild gebildet werden kann.

13. Einrichtung mit Strahlungsquantenzählung nach Anspruch 12, dadurch gekennzeichnet, daß die Halbleiterelemente aus einem Halbleitermaterial mit einer effektiven Atomzahl von über 30 hergestellt sind, und daß die Halbleiterelemente im wesentlichen linear auf dem Substrat angeordnet sind und jedes ein Paar von entsprechenden Mehrfachelektroden auf gegenüberliegenden Seiten jedes Halbleiterelements mit einem Abstand von 0,1—0,5 mm zwischen dem Elektrodenpaar aufweist, um dadurch die lineare Anordnung der strahlungsempfindlichen Elektroden zu bilden.

14. Einrichtung mit Strahlungsquantenzählung nach Anspruch 12, dadurch gekennzeichnet, daß die Halbleiterelemente aus einem Halbleitermaterial mit einem Energiebandabstand von über 1,3 eV hergestellt sind.

15. Einrichtung mit Strahlungsquantenzählung nach Anspruch 12, weiterhin gekennzeichnet dadurch, daß ein Strahlungsschild für die Strahlung mit einem Schlitz vorgesehen ist, um die Strahlen der Strahlung auf fächerförmige, in einer linearen Anordnung von strahlungsempfindlichen Elementen enthaltenden Ebene liegende Strählung zu begrenzen.

16. Einrichtung mit Strahlungsquantenzählung nach Anspruch 15, dadurch gekennzeichnet, daß die Strahlungsquelle, der Strahlungsschild und die lineare Anordnung von strahlungsempfindlichen Elementen als Verbundstruktur mit einer festen räumlichen Beziehung aufgebaut sind, die bezüglich des Körper beweglich gehalten ist.

17. Einrichtung mit Strahlungsquantenzählung nach Anspruch 15, dadurch gekennzeichnet, daß der Strahlungsschild zwischen der Strahlungsquelle und dem Körper angeordnet ist und daß der Strahlungsschild und die lineare Anordnung betriebsmäßig so bewegt werden, daß die lineare Anordnung auf einer Ebene liegt, die durch die Strahlungsquelle und den Schlitz definiert ist.

18. Einrichtung mit Strahlungsquantenzählung nach Anspruch 12, dadurch gekennzeichnet, daß die Strahlungsimpulszählzeit jedes einzelnen der strahlungsempfindlichen Elemente unter 30 m sec liegt.

19. Einrichtung mit Strahlungsquantenzählung nach Anspruch 12, dadurch gekennzeichnet, daß sie ferner eine Strahlungsempfindlichkeits-Einstellvorrichtung zum Einstellen oder Ausgleichen von wenigstens einem Teil des Ausgangs der strahlungsempfindlichen Elemente enthält.

20. Einrichtung mit Strahlungsquantenzählung nach Anspruch 12, dadurch gekennzeichnet, daß sie ferner eine Strahlungsempfindlichkeits-Einstellvorrichtung enthält, die durch einen Rechner gebildet wird, der mit wenigstens einem Teil der strahlungsempfindlichen Elemente verbunden ist, und die Ausgänge der strahlungsempfindlichen Elemente einzustellen oder auszugleichen.

21. Einrichtung mit Strahlungsquantenzählung nach Anspruch 12, dadurch gekennzeichnet, daß die Halbleiterelemente mit CdTe hergestellt werden.

22. Einrichtung mit Strahlungsquantenzählung nach Anspruch 12, dadurch gekennzeichnet, daß die Halbleiterelemente mit GaAs hergestellt werden.

**Revendications**

1. Méthode de radiographie à comptage quantique comprenant les étapes consistant à:

—émettre un rayonnement à partir d'une source de rayonnement,

—recevoir le rayonnement qui a pénétré dans le corps par un réseau linéaire d'éléments sensibles aux rayonnement, de façon à produire des signaux contenant une information radiographique, et

—modifier la relation spatiale relative entre la source de rayonnement et les éléments sensibles aux rayonnements d'une part et le corps d'autre part,

caractérisé en ce que:

—les éléments sensibles aux rayonnements sont constitués d'éléments en semiconducteur de manière à recevoir simultanément le rayonnement et détecter le nombre des quanta de rayonnement reçu et produire des impulsions correspondantes;

—les impulsions de sortie des éléments respectifs sensibles aux rayonnements sont amplifiés par des amplificateurs correspondants d'impulsions et comptées par des compteurs ultérieurs d'impulsions connectés à ceux-ci, afin de produire des données sur le nombre des répétitions des impulsions, cette donnée sur le nombre des répétitions des impulsions provenant des compteurs d'impulsions sont donnés à des moyens de mémoire,

—puis, après ledit changement de la relation spatiale relative entre la source de rayonnement et les éléments sensibles aux rayonnements d'une part et le corps d'autre part, les étapes indiquées ci-dessus sont répétées séquentiellement avec un changement progressif coopérative de la relation spatiale relative, d'où la production d'un réseau à deux dimensions de données sur le nombre des répétitions d'impulsions à partir desquelles une image dégradée peut être formée.

2. Méthode de radiographie à comptage quantique selon la revendication 1, dans lequel:

—les éléments en semiconducteur sont constitués d'un matériau semiconducteur ayant un numéro atomique effectif supérieur à 30, et

—les éléments en semiconducteur sont disposés de manière sensiblement linéaire sur un substrat et chacun comporte une paire d'électrodes multiples correspondantes sur les faces opposées de chaque élément en semiconducteur avec une distance de 0,1—0,5 mm entre la paire d'électrodes, d'où la constitution du réseau linéaire d'éléments sensibles aux rayonnements.

3. Méthode de radiographie à comptage quantique selon la revendication 1, dans laquelle:

—les éléments en semiconducteur sont constitués d'un matériau semiconducteur ayant un intervalle de bande d'énergie supérieur à 1,3 eV.

4. Méthode de radiographie à comptage quantique selon la revendication 1, qui comprend en outre:

—un écran contre les rayonnements pour ledit rayonnement, ayant une fente pour limiter les rayons du rayonnement à des rayons en forme d'éventail situés dans un plan de manière à contenir le réseau linéaire d'éléments sensibles aux rayonnements.

5. Méthode de radiographie à comptage quantique selon la revendication 5, dans laquelle:

—la source de rayonnement, l'écran contre les rayonnements et le réseau linéaire d'éléments sensibles aux rayonnements sont montés dans une construction composite ayant une relation spatiale fixe, qui est maintenue mobile par rapport au corps.

6. Méthode de radiographie à comptage quantique selon la revendication 4, dans laquelle:

—l'écran contre les rayonnements est disposé entre la source de rayonnement et le corps, et

—l'écran contre les rayonnements et le réseau linéaire sont mobiles d'une manière fonctionnelle telle que le réseau linéaire est dans un plan défini par la source de rayonnement et la fente.

7. Méthode de radiographie à comptage quantique selon la revendication 1, dans laquelle:

—le temps de comptage des impulsions de rayonnement de chacun des éléments sensibles aux rayonnements est inférieur à 30 ms.

8. Méthode de radiographie à comptage quantique selon la revendication 1, qui comprend en outre:

—un moyen d'ajustage de la sensibilité aux rayonnements afin d'ajuster ou de compenser au moins une partie de la sortie des éléments sensibles aux rayonnements.

9. Méthode de radiographie à comptage quantique selon la revendication 1, qui comprend en outre:

—un moyen d'ajustement de la sensibilité aux rayonnements qui est constitué par un moyen de calcul connecté à au moins une partie des éléments sensibles aux rayonnements afin d'ajuster ou de compenser les sorties des éléments sensibles aux rayonnements.

10. Méthode de radiographie à comptage quantique selon la revendication 1, dans laquelle:

—les éléments en semiconducteur sont constitués de CdTe.

11. Méthode de radiographie à comptage quantique selon la revendication 1, dans laquelle:

—les éléments en semiconducteur sont constitués de GaAs.

12. Appareil de radiographie à comptage quantique comprenant:

—une source de rayonnements pour émettre un rayonnement;

—un réseau linéaire d'éléments sensibles aux rayonnements pour recevoir un rayonnement qui pénètre dans un corps, d'où la production de signaux contenant une information radiographique,

—un moyen pour changer la relation spatiale relative entre la source de rayonnement et les éléments sensibles aux rayonnements d'une part et le corps d'autre part, caractérisé en ce que:

—les éléments sensibles aux rayonnements sont constitués d'éléments en semiconducteur de manière à recevoir simultanément le rayonnement et détecter le nombre de quanta de rayonnement reçus afin de produire des impulsions correspondantes,

—l'appareil radiographique à comptage quantique comprenant:

—des amplificateurs d'impulsions connectés respectivement à des éléments correspondants parmi les éléments sensibles aux rayonnements,

—des compteurs d'impulsions connectés respectivement à des amplificateurs correspondants parmi les amplificateurs d'impulsions, afin d'émettre une donnée sur le nombre des répétitions d'impulsions correspondantes à partir de ceux-ci,

—un moyen de mémoire pour stocker la donnée sur le nombre des répétitions d'impulsions, et

—un moyen de commande pour modifier la relation spatiale relative, d'où la production d'un réseau à deux dimensions de données sur le nombre des répétitions des impulsions à partir duquel une image dégradée peut être formée.

13. Appareil de radiographie à comptage quantique selon la revendication 12, dans lequel:

—les éléments en semiconducteur sont constitués d'un matériau semiconducteur ayant un numéro atomique effectif supérieur à 30, et

—les éléments en semiconducteur sont disposés d'une façon sensiblement linéaire sur un substrat et chacun comporte une pair d'électrodes multiples correspondantes sur les faces opposées de chaque élément en semiconducteur avec une distance de 0,1—0,5 mm entre la paire d'électrodes, d'où la constitution du réseau linéaire d'éléments sensibles aux rayonnements.

14. Appareil de radiographie à comptage quantique selon la revendication 12, dans lequel:

—les éléments en semiconducteur sont constitués d'un matériau semiconducteur ayant un intervalle de bande d'énergie supérieur à 1,3 eV.

15. Appareil de radiographie à comptage quantique selon la revendication 12, qui comprend en outre:

—un écran contre les rayonnements pour ledit rayonnement, ayant une fente pour limiter les rayons du rayonnement à des rayons en forme d'éventail situés dans un plan de manière à contenir le réseau linéaire d'éléments sensibles aux rayonnements;

16. Appareil de radiographie à comptage quantique selon la revendication 15, dans lequel:

—la source de rayonnement, l'écran contre les rayonnements et le réseau linéaire d'éléments sensibles aux rayonnements sont montés dans une construction composite en relation spatiale fixe, qui est maintenue de façon mobile en relation avec le corps.

17. Appareil de radiographie à comptage quantique selon la revendication 15, dans lequel:

—l'écran contre les rayonnements est disposé entre la source de rayonnement et le corps, et

—l'écran contre les rayonnements et le réseau linéaire se déplacent d'une manière fonctionnelle telle que le réseau linéaire se trouve dans un plan défini par la source de rayonnement et la fente.

18. Appareil de radiographie à comptage quantique selon la revendication 12, dans lequel:

—la période de comptage des impulsions de rayonnement de chacun des éléments sensibles aux rayonnements est inférieure à 30 ms.

19. Appareil de radiographique à comptage quantique selon la revendication 12, qui comprend en outre:

—un moyen de réglage de la sensibilité aux rayonnements afin d'ajuster ou de compenser au moins une partie de la sortie des éléments sensibles aux rayonnements.

20. Appareil de radiographie à comptage quantique selon la revendication 12, qui comprend en outre:

—un moyen d'ajustement de la sensibilité aux rayonnements qui est constitué par un moyen de calcul connecté à au moins une partie des éléments en semiconducteur pour rayonnement afin d'ajuster ou de compenser les sorties de éléments sensible aux rayonnements.

21. Appareil de radiographie à comptage quantique selon la revendication 12, dans lequel:

—les éléments en semiconducteur sont constitués de CdTe.

22. Appareil de radiographie à comptage quantique selon la revendication 12, dans lequel:

—les éléments en semiconducteur sont constitués de GaAs.

FIG.1

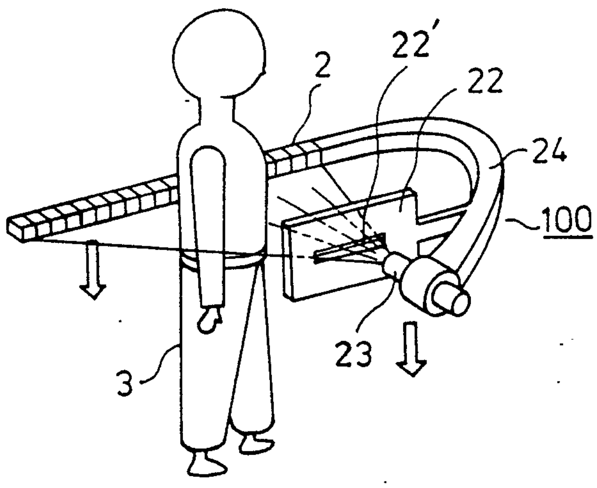


FIG.2

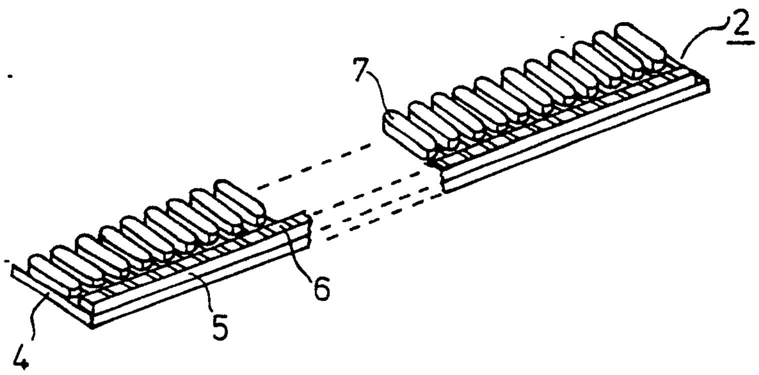

FIG.3

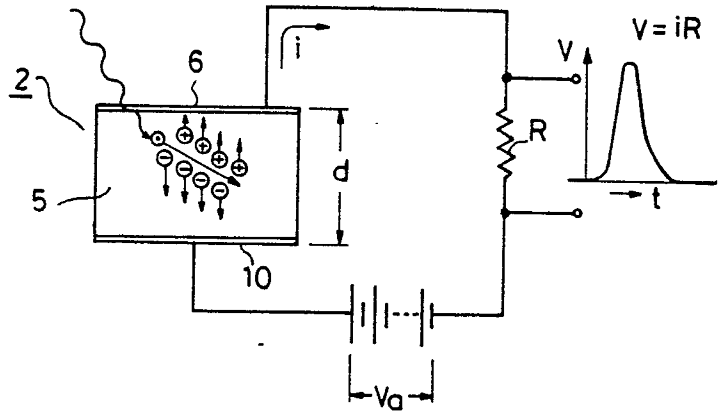


FIG.4 (a)

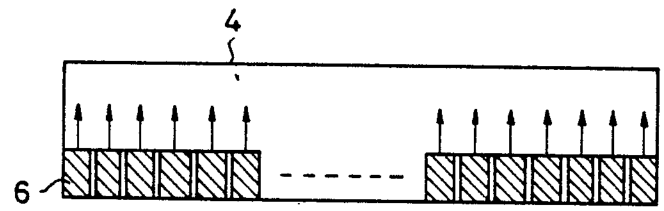


FIG.4 (b)

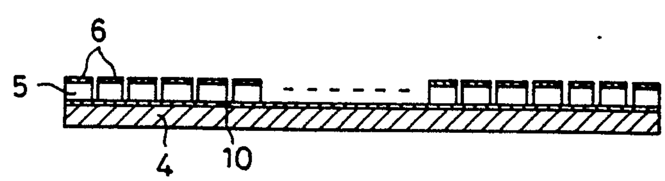

FIG.5

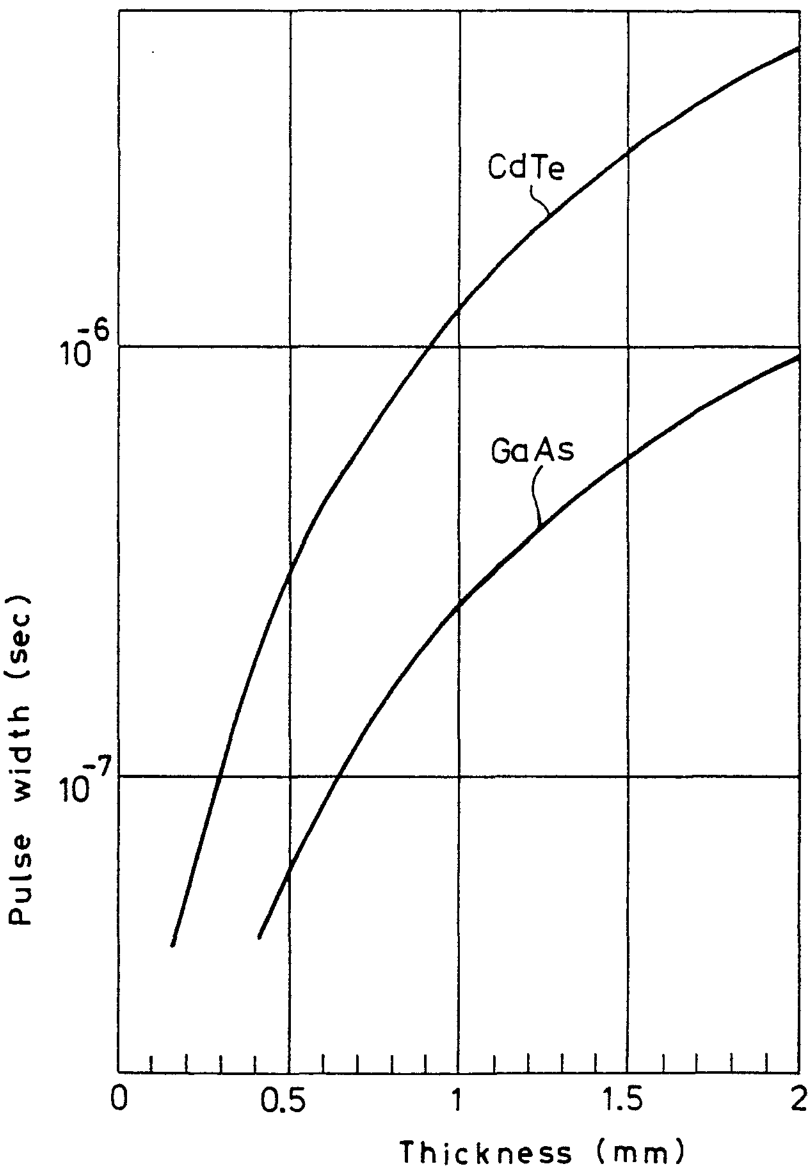

FIG.6

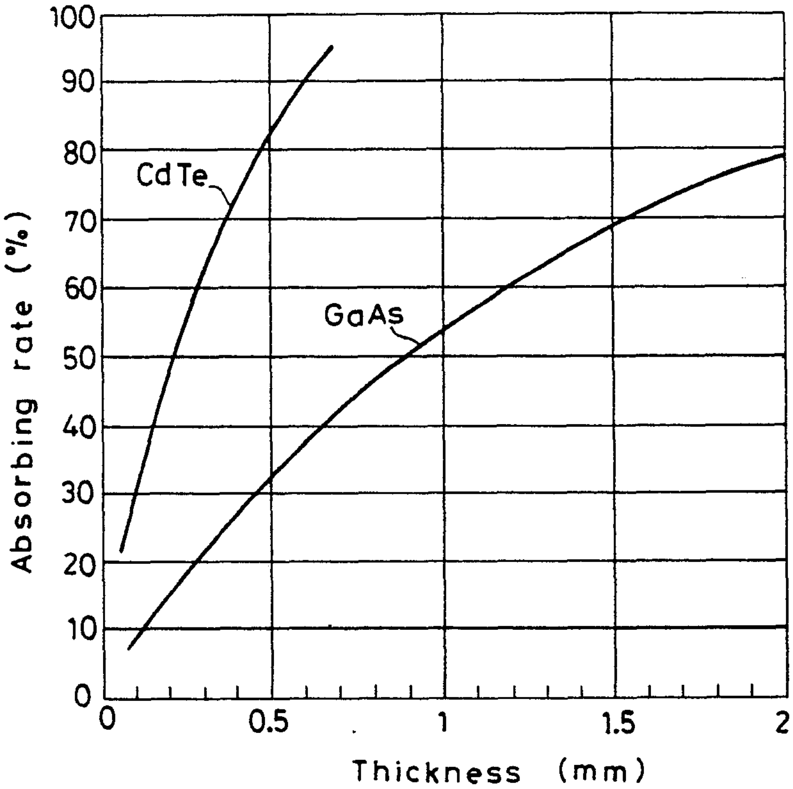
100
90
80
70
60
50
40
30
20
10
0
Absorbing rate (%)
CdTe
GaAs
0
0.5
1
1.5
2
Thickness (mm)

FIG.7(a)

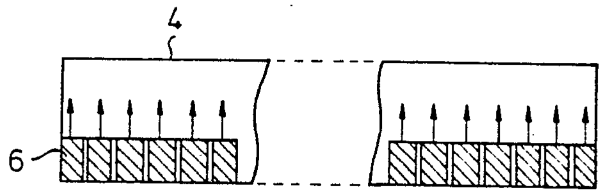


FIG.7(b)

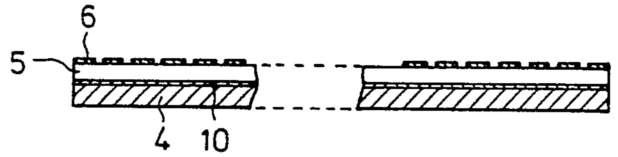


FIG.8

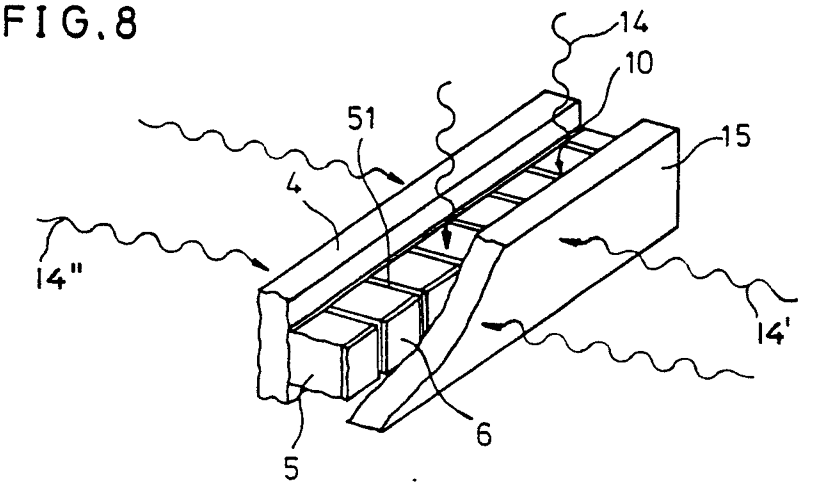

FIG. 9

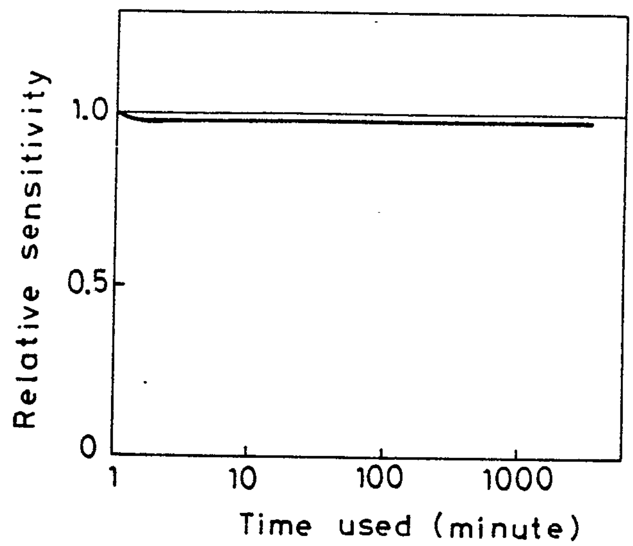
Relative sensitivity
1.0
0.5
0
1
10
100
1000
Time used (minute)

FIG. 10

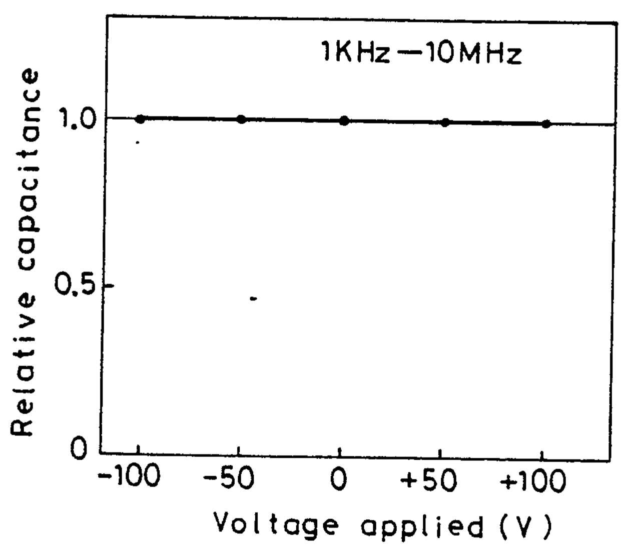
1KHz — 10MHz
Relative capacitance
1.0
0.5
0
-100
-50
0
+50
+100
Voltage applied (V)

FIG.11

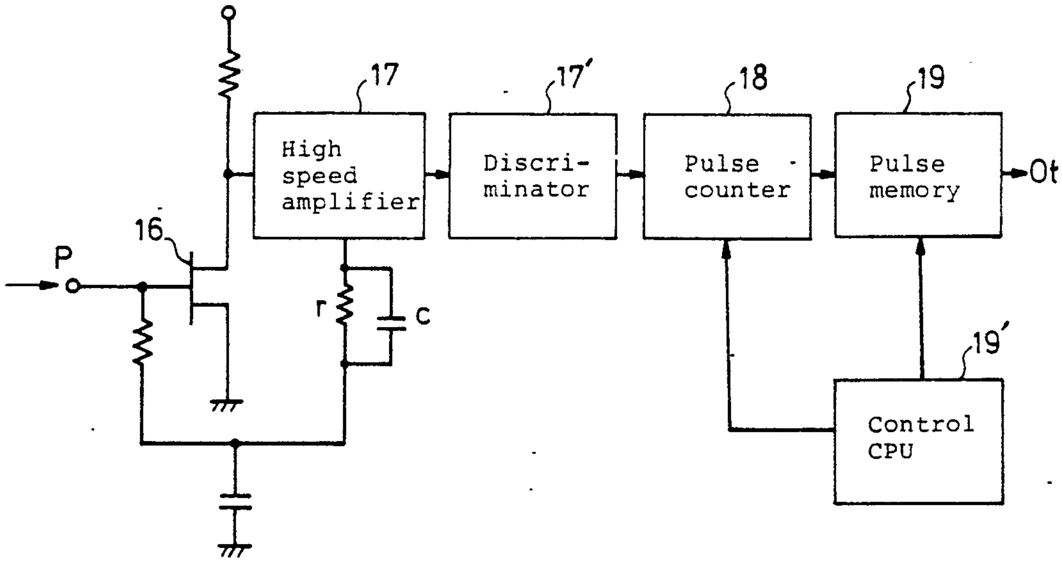
17
17'
18
19
High speed amplifier
Discri-minator
Pulse counter
Pulse memory
Ot
16
P
r
c
19'
Control CPU

FIG.12

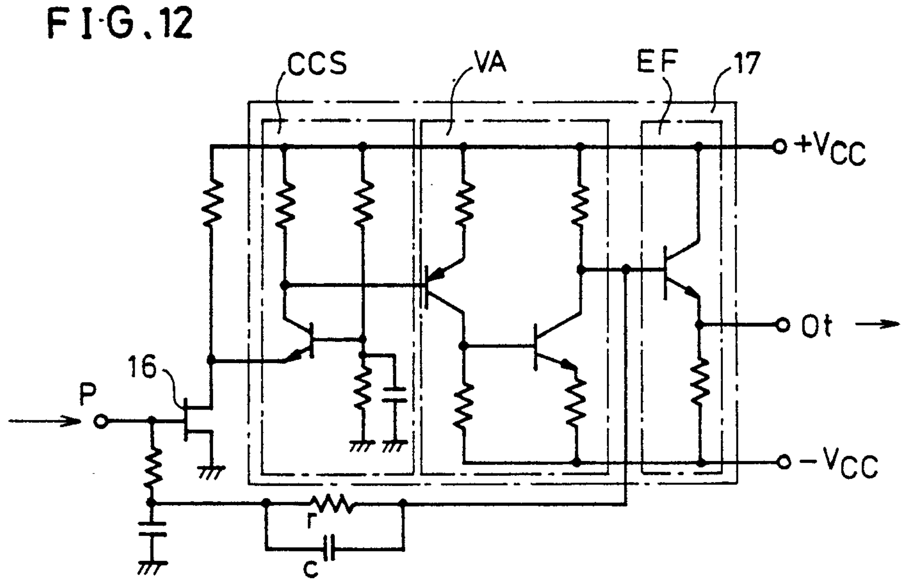
CCS
VA
EF
17
+VCC
Ot
16
P
-VCC
r
c

FIG.13(a)

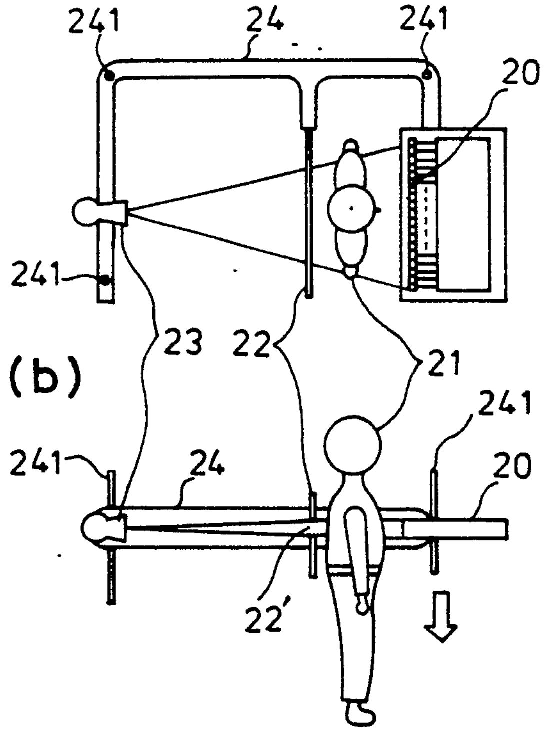


FIG.13(b)

FIG.14(a)

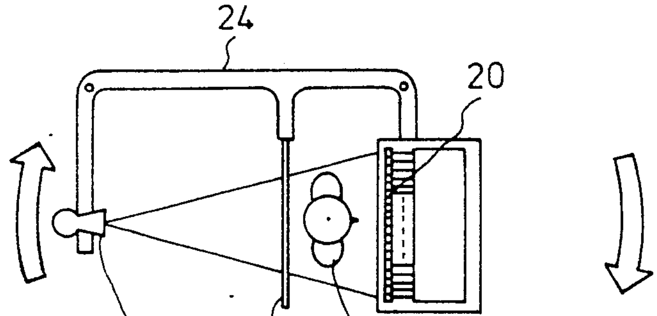


FIG.14(b)